# EUROPÄISCHE PATENTSCHRIFT

(11) **EP 3 437 559 B1**
(45) Veröffentlichungstag und Bekanntmachung des Hinweises auf die Patenterteilung: **24.06.2020**
(21) Anmeldenummer: 17184699.1
(22) Anmeldetag: 03.08.2017
(51) Int. Cl.: A61B 6/00, A61B 6/03, A61B 8/00, A61B 5/05

(54) **ERMITTELN EINES FUNKTIONSPARAMETERS BETREFFEND EINE LOKALE GEWEBEFUNKTION FÜR MEHRERE GEWEBEBEREICHE**
DETERMINATION OF A FUNCTIONAL PARAMETER RELATING TO A LOCAL TISSUE FUNCTION FOR MULTIPLE TISSUE AREAS
DÉTERMINATION D'UN PARAMÈTRE FONCTIONNEL RELATIF À UNE FONCTION TISSULAIRE LOCALE POUR DES RÉGIONS TISSULAIRES MULTIPLES

(43) Veröffentlichungstag der Anmeldung: 06.02.2019
(73) Patentinhaber: Siemens Healthcare GmbH, 91052 Erlangen (DE)
(72) Erfinder: Flohr, Thomas, 91486 Uehlfeld (DE); Krauß, Bernhard, 90559 Burgthann (DE); Schmidt, Bernhard, 90766 Fürth (DE); Sedlmair, Martin, 90513 Zirndorf (DE)

(56) Entgegenhaltungen:
- EP-A1- 2 810 598
- DE-A1-102013 220 018
- DE-A1-102015 221 877
- DE-B3-102013 218 047

## Beschreibung

Die klassische Tumortherapie umfasst neben der operativen Entfernung eines Tumors die Chemotherapie, bei der einem Patienten Zytotoxine oder Zytostatika verabreicht werden, welche ein gezieltes Absterben von Tumorzellen bewirken oder ein Tumorwachstum verhindern sollen. Sie umfasst auch die Strahlentherapie, bei der ein Tumor mit ionisierender oder Partikelstrahlung behandelt wird, was ebenfalls zum Absterben von Tumorzellen führen soll. Zur Ermittlung des Ansprechverhaltens eines Tumors wird häufig auf sogenannte RECIST-Kriterien zurückgegriffen. Gängige RECIST-Kriterien sind zum Beispiel ein maximaler Durchmesser (LAD) eines Tumors oder sein Kurzachsendurchmesser (SAD), also im Wesentlichen Angaben über die Größe des Tumors, gemessen zu einem bestimmten Betrachtungszeitpunkt. Ermittelt man anhand der RECIST-Kriterien über einen Behandlungszeitraum hinweg ein Verschwinden bzw. eine Verkleinerung des Tumors, insbesondere ohne das Erscheinen von neuen Läsionen, ist das ein Anzeichen für eine vollständigen (CR-Complete Response) bzw. teilweise (PR-Partial Response) Tumorantwort. Anders ausgedrückt, der Tumor spricht auf die eingesetzte Therapie an. Die Behandlung hat Erfolg. Wird über den Behandlungszeitraum hinweg eine gleichbleibende Tumorgröße ermittelt, ist das ein Anzeichen für eine unveränderte Erkrankung (SD-Stable Disease), was ebenfalls einem Behandlungserfolg entsprechen kann. Vergrößert sich der Tumor während der Behandlung bzw. kommen neue Läsionen hinzu, ist dies ein Anzeichen für eine fortschreitende Erkrankung (PD-Progressive Disease) und letztlich für einen Misserfolg der gewählten Behandlungsmethode.

Daneben gibt es modernere Tumorbehandlungsmethoden wie zum Beispiel die Anti-Angiogenese oder die Immuno-Therapie. Die Anti-Angiogenese ist darauf gerichtet, medikamentös die Blutversorgung von Tumorgewebe einzudämmen, indem die Gefäßbildung innerhalb des Tumors unterdrückt wird. Bei der Immuno-Therapie wird das körpereigene Immunsystem unterstützt, die Tumorzellen, die sich ansonsten einer körpereigenen Immunantwort entziehen würden, zu zerstören.

Die klassischen Behandlungsmethoden verursachen ein Gleichbleiben oder eine Verringerung der Tumorgröße. Neuartige Behandlungsmethoden haben jedoch oft zumindest für eine Übergangszeit keine Auswirkung auf die Tumorgröße oder können sogar ein Wachstum des Tumors bewirken. Erst bei länger anhaltender Behandlungsdauer zeigt sich die angestrebte Verkleinerung. Darüber hinaus können neuere Therapieformen strukturelle Veränderungen des Tumorgewebes verursachen, bspw. Nekrosen im Inneren des Tumors.

Nicht zuletzt aus wirtschaftlicher Sicht ist Zeit in der Krebstherapie ein kritischer Faktor, sodass das Ansprechverhalten auf eine gewählte Therapieform schnellstmöglich ermittelt werden sollte. Bei neuartigen Therapien erweisen sich aufgrund ihrer veränderten Wirkweise die üblichen Kriterien wie RECIST als ungenau oder sogar falsch, sodass diese für den vorgegebenen Zeitrahmen nicht oder nur eingeschränkt herangezogen werden können. Für die neuartigen Therapieverfahren werden daher Kriterien benötigt, die einen Rückschluss auf das Ansprechverhalten von Tumorzellen auf eine Behandlung, bspw. ihre lokale Blutversorgung, zulassen.

Es ist bekannt, unter Kontrastmittelgabe mittels quantitativer, medizinischer Bildgebungsverfahren wie zum Beispiel der Computertomographie (CT) mit zwei unterschiedlichen Energiespektren (Dual Energy) oder der dynamischen Perfusions-CT die Durchblutung bzw. das Blutvolumen in Tumorgewebe darzustellen, indem bspw. die im Tumorgewebe angereicherte Kontrastmittelmenge ermittelt wird. Bislang wird das Blutvolumen für die gesamte Läsion ausgewertet, sodass lokale Unterschiede der Blutversorgung oder lokale Veränderungen der Blutgefäßstruktur nicht berücksichtigt werden. Mit anderen Worten entspricht das derart ermittelte Blutvolumen einem mittleren Blutvolumen über die gesamte Läsion hinweg.

Das Dokument EP 2810598 A1 offenbart ein Verfahren zur Unterstützung einer Operation, bei welchem in einer Zielgeweberegion ein abnormaler Bereich extrahiert wird und für den abnormalen Bereich ein Exzisionsverfahren ermittelt wird.

Eine manuelle Definition bzw. Auswahl von interessierenden Bereichen (ROIs) zur Analyse eines lokalen Blutvolumens kann die lokalen Unterschiede zwar aufzeigen, ist jedoch stark Nutzer-abhängig und dadurch schlecht reproduzierbar und grundsätzlich fehleranfällig.

Alternative Analyse-Verfahren wie beispielsweise bekannte Texturanalyse-Verfahren wären unter Auswertung der strukturellen Eigenschaften des abgebildeten Tumorgewebes im Grunde in der Lage, sog. ,Perfusion Maps', also mehrdimensionale Durchblutungskarten für einen Tumor zu erzeugen, reagieren jedoch sehr empfindlich auf kleinste Änderungen in der Bildverarbeitungskette (Rekonstruktionskern, Schichtdicke, Rauschfilter, etc). Eine Vergleichbarkeit von Initial- und Kontrollmessung wäre auch hier nicht gegeben.

Demgegenüber ist es Aufgabe der vorliegenden Erfindung, alternative Mittel bereit zu stellen, die es erlauben, zuverlässig und reproduzierbar Rückschlüsse auf eine lokale Gewebefunktion abzuleiten. Insbesondere ist es Aufgabe der vorliegenden Erfindung, frühzeitig nach Therapiebeginn Information über eine lokale Durchblutung von Tumorgewebe abzuleiten.

Diese Aufgabe wird gelöst durch ein Verfahren zum Bestimmen einer Gewebefunktion eines Gewebes, entsprechende Recheneinheit und medizinische Bildgebungsanlage, entsprechendes Computerprogramm und entsprechenden computerlesbaren Datenträger gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zum Bestimmen einer lokalen Gewebefunktion eines Gewebes in einer interessierenden Körperregion eines Untersuchungsobjektes. Das Verfahren umfasst eine Vielzahl von Schritten.

Das erfindungsgemäße Verfahren zielt darauf ab, eine Gewebefunktion eines Gewebes in einer interessierenden Körperregion eines Untersuchungsobjektes zu ermitteln. Dabei legt das Verfahren besonderes Augenmerk auf die individuelle Ermittlung dieser Gewebefunktion für lokale Bereiche des Gewebes. Mit anderen Worten erlaubt das Verfahren eine Darstellung lokaler Unterschiede in der Gewebefunktion.

Eine Gewebefunktion im Sinne dieser Erfindung bezeichnet eine physikalische, chemische, funktionelle und/oder strukturelle Eigenschaft eines Gewebes, wie zum Beispiel eine Materialdichte, einen Stoff- bzw. Substanzgehalt oder -anteil, eine Substanz-Anreicherungsrate (bei dynamischen Messungen) oder dergleichen.

Unter einem Gewebe ist im Sinne der Erfindung eine Vielzahl gleichartig ausgebildeter, in der Regel zusammenhängender Körperzellen zu verstehen, die eine gleiche bzw. ähnliche Funktion ausüben. Mit anderen Worten entspricht ein Gewebe einem Gewebetyp oder einer Gewebeart, wobei das Gewebe ein Tumorgewebe ist. Ein Gewebe im Sinne der Erfindung kann auch eine Läsion unbekannter Beschaffenheit mit Vermutung auf Tumorgewebe, also eine anomale Veränderung, umfassen oder bilden. Ein Gewebe im Sinne der Erfindung kann nur einen oder mehrere Gewebetypen umfassen.

Das Gewebe befindet sich in einer interessierenden Körperregion. Diese entspricht im Sinne der hiesigen Erfindung dem zu untersuchenden Körperteil bzw. der zu untersuchenden Körperregion, bspw. dem Abdomen oder dem Schädel. In diesem Sinne entspricht die zu untersuchende Körperregion dem Teil eines Untersuchungsobjektes, welches mittels einer medizinischen Bildgebungsanlage, zum Beispiel eines Computertomographen, untersucht bzw. abgebildet werden soll.

Insofern wird im Folgenden ohne Beschränkung der Allgemeinheit von einem Patienten als Untersuchungsobjekt ausgegangen, wobei es sich meist um einen Menschen handelt. Grundsätzlich kann der Patient auch ein Tier sein. Im Folgenden werden daher die beiden Begriffe "Untersuchungsobjekt" und "Patient" synonym verwendet. Das Untersuchungsobjekt kann alternativ eine Pflanze oder ein nicht-lebender Gegenstand, z.B. ein historisches Artefakt oder dergleichen sein.

Das Segmentieren einer Außenkontur des Gewebes anhand von wenigstens einer medizinischen Bildaufnahme darstellend die interessierende Körperregion des Untersuchungsobjektes umfassend das Gewebe entspricht einem ersten Verfahrensschritt.

Eine medizinische Bildaufnahme ist eine mehrdimensionale, insbesondere eine zwei-dimensionale oder drei-dimensionale Abbildung von zu der interessierenden Körperregion gehörigen Sensor-Daten, die mittels einer medizinischen Bildgebungsanlage erzeugt wurden. Die medizinische Bildaufnahme kann eine Computer-Tomographie-Aufnahme, MRT-Aufnahme, Röntgenaufnahme, Positronen-Emissions-Tomographie(PET)-Aufnahme, Röntgen-C-Bogen-Aufnahme, Ultraschall-Aufnahme, Single-Photon-Emissions-Tomographie (SPECT)-Aufnahme oder dergleichen sein. Die medizinische Bildaufnahme kann eine Einzelaufnahme, aber auch Teilobjekt einer mehrschichtigen Aufnahmeserie sein.
Die Dimension der Bildaufnahmen gibt an, ob eine ausgewählte Schicht mit einer definierten Schichtdicke (2D) der interessierenden Region oder ein ausgewähltes Volumen (3D) dargestellt ist. Im Falle mehrerer medizinischer Bildaufnahmen kann die Abbildung des Gewebes drei- oder vier-dimensional sein, dann jeweils mit oder ohne Zeitverlauf (3D oder 4D). Bei medizinischen Bildaufnahmen mit Zeitverlauf handelt es sich in der Regel um funktionelle bzw. dynamische Bildaufnahmen, zum Beispiel um eine Perfusionsmessung. Im Falle mehrerer medizinischer Bildaufnahmen können auch Einzelaufnahmen verschiedener Energiespektren umfasst sein. Eine medizinische Bildaufnahme kann auch ein konstruiertes bzw. zusammengesetztes Bild sein, welches aus mehreren Einzelaufnahmen, insbesondere aus verschiedenen Aufnahmetechniken, zusammengesetzt wurde bzw. welches Bildinformation aus mehreren Einzelaufnahmen umfasst. Mit anderen Worten kann eine medizinische Bildaufnahme als wenigstens eine Aufnahme aus der folgenden Gruppe von Aufnahmen ausgebildet sein: ein zweidimensionales, tomographisches Bild, ein dreidimensionales Bild, ein vierdimensionales Bild oder ein multi-spektrales Bild.

Die Bilderfassung kann zeitlich mit der Durchführung des erfindungsgemäßen Verfahrens korrelieren. Alternativ kann die medizinische Bildaufnahme zu einem beliebigen Zeitpunkt vor der Ausführung des Verfahrens erfasst worden sein.

Gemäß diesem ersten Schritt erfolgt ein Segmentieren der Außenkontur des betrachteten Gewebes in der medizinischen Bildaufnahme. Mit anderen Worten, wird das zu untersuchende Gewebe nach außen hin abgegrenzt und seine Außenkontur definiert. Die Außenkontur kann eine in Abhängigkeit der Größe des bei der Bilderfassung gewählten Sichtfeldes (FoV) offene oder geschlossene Linie (2D) oder Fläche (3D) sein. Diese trennt das Gewebe von umliegenden Strukturen. Das Segmentieren kann automatisch, semi-automatisch anhand in Fachkreisen bekannter Segmentierungsalgorithmen oder manuell in der Bildaufnahme durch einen Benutzer durchgeführt werden.

Einen zweiten Schritt des erfindungsgemäßen Verfahrens bildet das Unterteilen des segmentierten Gewebes in wenigstens zwei Gewebebereiche. Mit anderen Worten werden für das Gewebe wenigstens zwei Teilbereiche gebildet und innerhalb der segmentierten Außenkontur des Gewebes angeordnet, die derart eine Substruktur des Gewebes darstellen. Die Anzahl sowie die Anordnung der Gewebebereiche zu einander sind variabel und können insbesondere von der Größe bzw. der Art des untersuchten Gewebes abhängen. Alternativ oder zusätzlich können Anzahl und Lage der Gewebebereiche in Abhängigkeit der Wahl des im Folgenden zu ermittelnden Funktionsparameters bestimmt werden. Die Unterteilung erfolgt bevorzugt automatisch, kann aber auch semi-automatisch erfolgen, zum Beispiel, indem dem Nutzer zunächst ein Vorschlag oder eine Auswahl von Vorschlägen angezeigt wird, den bzw. die dieser bestätigen oder auswählen kann. Die Unterteilung erfolgt weiter bevorzugt basierend auf Erfahrungs- bzw. Referenzwerten. Mit anderen Worten können zum Beispiel in einer Datenbank Standard-Unterteilungen des segmentierten Gewebes für bestimmte Gewebearten, Gewebegrößen, betrachtete Funktionsparameter und/oder dergleichen hinterlegt sein, die als Referenz bzw. Referenzwerte in die Unterteilung einfließen bzw. dabei berücksichtigt werden. Bevorzugt fließen in die Definition der Lage und/oder Größe der Gewebebereiche, sofern bekannt, Informationen, Erfahrungswerte bzw. Annahmen über eine räumliche Verteilung des im Folgenden zu ermittelnden Funktionsparameters mit ein. Derart stellt das erfindungsgemäße verfahren vorteilhaft sicher, dass lokale Schwankungen der Gewebefunktion dargestellt werden.

In einem dritten Schritt wird, wie oben bereits angedeutet, ein Funktionsparameter betreffend die Gewebefunktion für jeden der wenigstens zwei Gewebebereiche ermittelt. Der Funktionsparameter ist ein Maß, ein Repräsentant bzw. eine Kenngröße für die Gewebefunktion. Mit anderen Worten kennzeichnet ein Funktionsparameter eine bestimmte Gewebefunktion bzw. lässt Rückschlüsse auf diese Gewebefunktion zu. Das Ermitteln umfasst erfindungsgemäß eine quantitative und/oder qualitative Auswertung von in den medizinischen Bildaufnahmen enthaltener Bildinformation, jeweils bezogen auf die einzelnen Gewebebereiche. Zum Beispiel kann bei einer CT-Bildaufnahme unter Kontrastmittelgabe für jeden Gewebebereich eine Auswertung der Helligkeitswerte der Bildelemente (HU-Werte) erfolgen, sodass auf ein (mittlere) Durchblutung des Gewebes in jedem der Gewebebereiche zurückgeschlossen werden kann.

Alternativ können auch mehr als nur ein Funktionsparameter pro Gewebebereich ermittelt werden. Insofern können mit dem erfindungsgemäßen Verfahren mehrere Gewebefunktionen parallel untersucht werden.

Zusammenfasend haben die Erfinder also erkannt, dass das erfindungsgemäße Verfahren ermöglicht, räumliche Schwankungen bzw. Unterschiede einer Gewebefunktion darzustellen, indem ein für eine Gewebefunktion charakteristischer Funktionsparameter individuell für verschiedene, also wenigstens zwei Gewebebereiche bestimmt wird. Die derart für die verschiedenen Gewebebereiche ermittelten, lokalen Werte des Funktionsparameters dienen also Maß für die Homogenität bzw. Inhomogenität einer Gewebefunktion über das untersuchte Gewebe hinweg. Das erfindungsgemäße Verfahren eignet sich damit für eine Vielzahl von verschiedenen Anwendungen in der Medizin.

Das erfindungsgemäße Verfahren eignet sich insbesondere zur Anwendung bei der Untersuchung des Ansprechverhaltens von Tumorerkrankungen, zur Überwachung eines beliebigen anderen Krankheitsverlaufs, wie bspw. Osteoporose, oder aber allgemeiner auch für die Durchführung von Kontrolluntersuchungen zu einem späteren Zeitpunkt, denn die Unterteilung des segmentierten Gewebes sowie das erfindungsgemäße Ermitteln des Funktionsparameters, wie es bei einer initialen Erstuntersuchung vorgenommen wurde, kann auf Bildaufnahmen einer späteren Kontrollmessung robust und reproduzierbar übertragen werden und liefert so zu der Erstuntersuchung vergleichbare Daten.

Es sei abschließend nochmals erwähnt, dass die beschriebenen Verfahrensschritte, also das Segmentieren, das Unterteilen sowie das Ermitteln in den medizinischen Bildaufnahmen pixel- und/oder voxel-basiert erfolgen können.

Gemäß der vorliegenden Erfindung sind die wenigstens zwei Gewebebereiche derart schichtartig angeordnet, dass ein erster Gewebebereich einen zweiten Gewebebereich vollständig umschließt. Mit anderen Worten werden die Gewebebereiche ringartig bzw. verschachtelt angeordnet, sodass lediglich ein äußerer Gewebebereich äußerlich an der segmentierten Außenkontur des Gewebes anliegt bzw. diese einschließt und innerlich die übrigen Gewebebereiche umfasst. Diese Anordnung entspricht einem zwiebelschicht-artigen Aufbau. Sie eignet sich besonders für die Untersuchung von Gewebefunktionen von Tumorgewebe bzw. von Läsionen unbekannter Beschaffenheit mit Vermutung auf Tumorgewebe, denn diese weisen häufig sowohl strukturelle als auch funktionelle Unterschiede zwischen Kern- und Randbereichen auf. Diese Unterschiede können mit der vorgeschlagenen Anordnung der Gewebebereiche besonders vorteilhaft berücksichtigt bzw. aufgelöst werden.

Alternative Anordnungen, wie bspw. eine quadranten-artige oder schicht-artige Einteilung der Gewebebereiche, sind ebenfalls denkbar und im Sinne der Erfindung, sofern sie sich für den konkreten Anwendungsfall als geeignet erweisen.

Gemäß einem weiteren Aspekt der vorliegenden Erfindung weist die Außenkontur der wenigstens zwei Gewebebereiche jeweils die gleiche Form wir die Außenkontur des segmentierten Gewebes auf. Dies bedeutet, dass der Verlauf der Außenkontur eines jeden Gewebebereichs morphologisch identisch zum Verlauf der Außenkontur des segmentierten Gewebes ist. Lediglich die Größe der Außenkonturen unterscheidet sich. Dies entspricht einer besonders bevorzugten Variante der Erfindung, denn derart ist eine Unterteilung in die Gewebebereiche besonders einfach, da die Form bzw. der Verlauf der Außenkonturen mit der Segmentierung des Gewebes bereits festgelegt ist. Diese Vorgehensweise findet insbesondere dann Anwendung, wenn die Außenkontur in der medizinischen Bildaufnahme mittels Segmentierung eindeutig definiert werden kann.

Insofern entspricht es einem weiteren besonders bevorzugten Aspekt der Erfindung, dass das Unterteilen des segmentierten Gewebes unter Verwendung einer morphologischen Operation erfolgt. Morphologische Operationen sind in Fachkreisen als Bildbearbeitungsmechanismen bekannt. Es handelt sich dabei um Operationen, die in der Regel mit dem Ziel auf die Gestalt eines Objektes angewandt werden, Veränderung dieser Gestalt zu bewirken, Störungen wie sie bspw. nach einer Segmentierung auftreten, zu beseitigen, bestimmte Formmerkmale zu berechnen oder bestimmte Formen in einem Bild zu detektieren. Das Objekt entspricht vorliegend dem untersuchten Gewebe, dessen Außenkontur bereits segmentiert wurde. Die segmentierte Außenkontur entspricht dabei der Gestalt des Objektes. Das Objekt sollte für die Bearbeitung mittels morphologischem Operator vorteilhaft als Binärbild vorliegen. Zur Bestimmung der Verläufe von Außenkonturen von einer Vielzahl von Gewebebereichen kann vorliegend bspw. ein Erosionsoperator angewandt werden, um eine gewünschte Verkleinerung der segmentierten Außenkontur zu erzielen. Die derart erzeugten Konturverläufe für die einzelnen Gewebebereiche können dann bspw. mittels Bildaddition oder Überlagerung in die medizinische Bildaufnahme übertragen bzw. integriert werden.

Diese Gestaltung bzw. Einteilung und Anordnung der Gewebebereiche entsprechend der Form bzw. des Verlaufs der segmentierten Außenkontur des Gewebes stellt eine bestmögliche Berücksichtigung der Geometrie des Gewebes.

Gemäß einem anderen bevorzugten Aspekt weisen die Außenkonturen der wenigstens zwei Gewebebereiche einen Abstand zu einander auf, der im Bereich von 0,2 mm bis 2,0 mm liegt. Mit anderen Worten sind die Außenkonturen von zwei benachbart angeordneten Gewebebereichen jeweils wie oben angegeben beabstandet. Wie eingangs bereits erwähnt, hängt die konkrete Lage und Größe der Gewebebereiche von verschiedenen Faktoren des einzelnen Untersuchungsobjektes ab, wie bspw. Größe des Gewebes, Struktur des Gewebes, Fragestellung der Untersuchung, auszuwertender Funktionsparameter oder dergleichen. Jedoch hat sich in der Praxis gezeigt, dass Gewebebereiche mit einer radialen Ausdehnung in einem Bereich zwischen 0,2 mm und 2,0 mm besonders gute Ergebnisse im Hinblick auf eine räumliche Inhomogenität einer Gewebefunktion liefern.

Gemäß einem weiteren bevorzugten Aspekt der vorliegenden Erfindung umfasst das segmentierte Gewebe eine medizinische Läsion. Mit anderen Worten kann das segmentierte Gewebe gesundes und Gewebe einer Läsion umfassen. Alternativ und entsprechend einem Hauptanwendungsfall der vorliegenden Erfindung bildet ausschließlich eine Läsion das segmentierte Gewebe. In einem weiteren Beispiel der vorliegenden Erfindung umfasst das segmentierte Gewebe keine Läsion. Im Sinne der Erfindung ist mit Läsion eine räumlich ausgedehnte Struktur zu verstehen, die im medizinischen Sinne auffällig ist, wie bspw. untypische bzw. unerwartete Abbildungseigenschaften bei einer medizinischen Bildgebung und/oder eine atypische Funktion, bspw. eine veränderte metabolische Aktivität. Bei einer Läsion kann es sich insbesondere um einen Tumor handeln, andere und insbesondere krankhafte Läsionen sind jedoch ebenfalls umfasst.

Gemäß einem anderen Aspekt der vorliegenden Erfindung ist der Funktionsparameter wenigstens ein Parameter aus der folgenden Gruppe von Parametern: Blutfluss, Iod-Anreicherung, Eisenanreicherung, Calcium-Dichte, Fettanteil.

Die drei erstgenannten Funktionsparameter lassen sich besonders gut ermitteln, wenn als medizinische Bildaufnahme eine CT-Perfusions-Aufnahme verwendet wird. Bei der CT-Perfusion wird mit Hilfe von Kontrastmittel, insbesondere Iod- oder Eisenhaltigem Kontrastmittel, und einer speziellen Post-Processing Software die Durchblutung von Gewebe, bspw. des Gehirns, der Leber oder des Herzens gemessen. Die interessierende Körperregion wird bei der CT-Perfusion nach der intravenösen Kontrastmittelinjektion widerholt über einen bestimmten Zeitraum, bspw. 40 s, hinweg gescannt, woraus ein 4-dimensionaler Bilddatensatz resultiert, der Aufschluss über Anflutung und Abflutung des Kontrakstmittels gibt.

Neben dem Blutfluss, der angibt, wie viel Volumen Blut (ml) pro Masse Gewebe (g) pro Zeit (min) fließt, können auch weitere Parameter, wie zum Beispiel das Blutvolumen, also, wie viel Volumen Blut (ml) pro Masse Gewebe (g) vorzufinden ist, oder eine Gewebe-Permeabilität also, wie viel Volumen Blut (ml) pro Masse Gewebe (g) pro Zeit (min) in das interessierende Gewebe gelangt, ausgewertet und bspw. farblich in einer Color-Map dargestellt werden.

Funktionsparameter wie Calcium-Dichte oder Fettanteil lassen sich bevorzugt basierend auf spektral aufgelösten medizinischen Bildaufnahmen mittels der in Fachkreisen bekannten Methode der Materialzerlegung ableiten. Spektral aufgelöste Bildaufnahmen können dabei insbesondere mittels multispektraler Computertomographie, bspw. mit Dual Source Tomographie, erzeugt werden.

Das erfindungsgemäße Verfahren liefert über die Auswertung eines der genannten Funktionsparameter insbesondere Aufschluss über die räumliche Verteilung und Inhomogenität einer Blutversorgung des untersuchten Gewebes, welche insbesondere Rückschlüsse auf eine Antwort bzw. Reaktion des Gewebes auf eine durchgeführte Therapiemaßnahme zulässt.

Gemäß einem weiteren Aspekt der Erfindung ist der Funktionsparameter wenigstens ein für Texturanalyse typischer Parameter aus der folgenden Gruppe von Parametern ist: mittlere Dichte, Moment, Heterogenität, Entropie, fraktale Dimension.

Texturanalyse im Sinne der Erfindung umfasst generell eine Charakterisierung bestimmter Regionen bzw. Bereiche einer medizinischen Bildaufnahme nach ihrem Texturgehalt, mit anderen Worten wertet eine Texturanalyse eine Funktion einer räumlichen Verteilung bzw. Veränderung von Pixel- oder Voxel-Intensitätswerten aus. Beispielsweise entspricht eine Texturanalyse einer quantitativen Messung von Größen wie Entropie, Wölbung oder Schiefe, die in Bezug auf eine in der Bildaufnahme dargestellte Oberfläche aus den genannten Intensitätswerten abgeleitet werden. Texturen in medizinischen Bildaufnahmen können, wie oben zum Teil schon erwähnt, bspw. unterschiedlichen (mittleren) Gewebedichten, verschiedenen Geweben bzw. Gewebetypen, verschiedenen Oberflächeneigenschaften oder dergleichen sein. Texturanalyse wird typischerweise in wenigstens einem Farbkanal, bspw. eines Graustufenbildes oder einem roten, grünen und/oder blauen Farbkanal einer medizinischen Bildaufnahme durchgeführt. Ein Farbkanal kann dabei insbesondere eine spezielle Aufnahmetechnik charakterisieren, bspw. verschiedene Spektren detektierter Strahlung.

Insofern kann der gemäß diesem Aspekt ermittelte Funktionsparameter eine Texturmetrik im Sinne einer Pixelintensitätsmetrik, einer Pixel-Varianzmetrik, einer Pixel-Korrelationsmetrik, einer Metrik bezüglich einer räumlichen Veränderung und/oder einer Metrik bezüglich einer Frequenz-Änderung sein.

Gemäß einem besonders bevorzugten Aspekt der vorliegenden Erfindung umfasst das Ermitteln eines Funktionsparameters eine Texturanalyse, welche wiederum umfasst, basierend auf Intensitätswerten von Bildelementen der medizinischen Bildaufnahme einen Merkmalsvektor zu bestimmen, ein maschinelles Lernverfahren auf den Merkmalsvektor anzuwenden und den Funktionsparameter als Ergebnis des maschinellen Lernverfahrens zu erzeugen.

Mit anderen Worten sieht das erfindungsgemäße Verfahren vor, Algorithmen des maschinellen Lernens auf Bildelemente, also Pixel oder Voxel der medizinischen Bildaufnahme, anzuwenden, um den Funktionsparameter zu bestimmen. Beispielsweise kann ein Algorithmus vor einer Anwendung anhand von Trainingsbildern mit oder ohne Unterstützung eines Nutzers trainiert werden, um spezielle Merkmale zu erkennen und einen Funktionsparameter abzuleiten. Bei dem maschinellen Lernverfahren kann es sich bspw. um ein in Fachkreisen bekanntes künstliches neuronales Netz, eine Deep Belief Verfahren oder dergleichen handeln.

Gemäß einem weiteren Aspekt der Erfindung ist die medizinische Bildaufnahme eine Bildaufnahme aus der folgenden Gruppe von Bildaufnahmen: Single-Energy-Aufnahme, multi-spektrale Computertomographie-Aufnahme, Perfusions-Computertomographie-Aufnahme, Ultraschall-Aufnahme, Magnetresonanz-Aufnahme und Perfusionsmagnetresonanz-Aufnahme oder dergleichen. Je nach Art der Bildaufnahme kann, wie eingangs bereits erwähnt, eine Kontrastmittelgabe in die Datenakquise involviert sein.

Die Erfindung betrifft ferner eine Recheneinheit zum Bestimmen einer Gewebefunktion eines Gewebes in einer interessierenden Region eines Untersuchungsobjektes aufweisend Mittel zum Durchführen des erfindungsgemäßen Verfahrens.

Die Erfindung betrifft auch eine medizinische Bildgebungsanlage mit einer erfindungsgemäßen Recheneinheit. Vorteilhaft ist die Recheneinheit in die medizinische Bildgebungsanlage integriert. Alternativ kann die Recheneinheit auch entfernt bzw. abgelegen angeordnet sein. Die Recheneinheit kann ausgebildet sein, insbesondere den Schritt des Ermittelns eines Funktionsparameters betreffend die Gewebefunktion für jeden von wenigstens zwei Gewebebereichen, aber auch das gesamte erfindungsgemäße Verfahren, für eine medizinische Bildgebungsanlage oder für eine Vielzahl von Anlagen durchzuführen, z.B. in einem Radiologie-Zentrum oder Krankenhaus umfassend mehrere Magnetresonanz-Anlagen.
Die Erfindung betrifft ferner ein Computerprogramm mit Programmcode, um das erfindungsgemäße Verfahren zum Bestimmen einer Gewebefunktion eines Gewebes in einer interessierenden Region eines Untersuchungsobjektes durchzuführen, wenn das Computerprogramm auf einem Computer ausgeführt wird.

Die Erfindung betrifft ferner einen computerlesbarer Datenträger mit Programmcode eines Computerprogramms, um das erfindungsgemäße Verfahren zum Bestimmen einer Gewebefunktion eines Gewebes in einer interessierenden Region eines Untersuchungsobjektes, wenn das Computerprogramm auf einem Computer ausgeführt wird. Vorteilhaft kann insbesondere das Ermitteln eines Funktionsparameters betreffend die Gewebefunktion für jeden von wenigstens zwei Gewebebereichen auf einem Computer, beispielsweise in einer Recheneinheit einer medizinischen Bildgebungsanlage, ausgeführt werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht einer medizinischen Bildgebungsanlage in Form eines Computertomographen gemäß einer Ausführungsform der vorliegenden Erfindung,
- FIG 2: eine Läsion in einer medizinischen Bildaufnahme gemäß dem Stand der Technik,
- FIG 3: eine andere Läsion in einer medizinischen Bildaufnahme gemäß dem Stand der Technik,
- FIG 4: eine Läsion in einer medizinische Bildaufnahme gemäß einem Ausführungsbeispiel der vorliegenden Erfindung,
- FIG 5: eine andere Läsion in einer medizinische Bildaufnahme gemäß einem Ausführungsbeispiel der vorliegenden Erfindung,
- FIG 6: eine schematische Darstellung eines erfindungsgemäßen Verfahrens gemäß einem Ausführungsbeispiel der vorliegenden Erfindung.

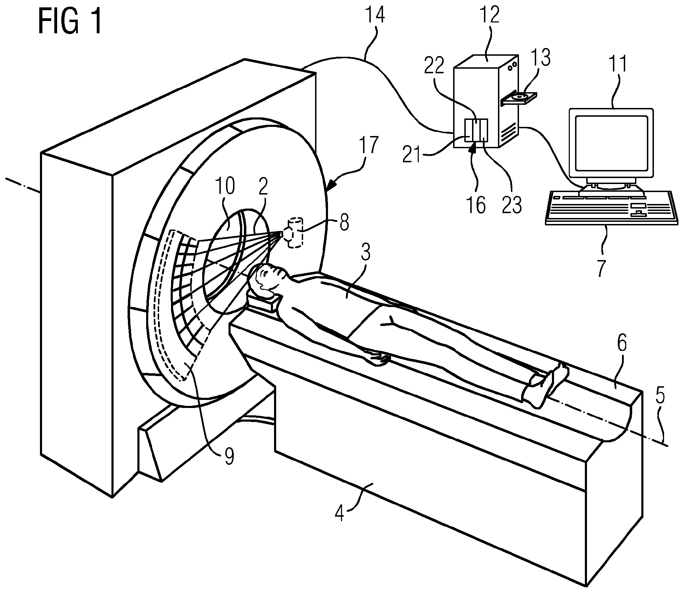

**Figur 1** zeigt eine medizinische Bildgebungsanlage in Form eine Computertomographen. Der hier gezeigte Computertomograph verfügt über eine Aufnahmeeinheit 17, umfassend eine Röntgenstrahlungsquelle 8 sowie einen Röntgenstrahlungsdetektor 9. Die Aufnahmeeinheit 17 rotiert während der Aufnahme von Röntgenprojektionen um eine Systemachse 5, und die Röntgenquelle 8 emittiert während der Aufnahme Röntgenstrahlen 2.

Ein Patient 3 liegt bei der Aufnahme von Röntgenprojektionen auf einer Patientenliege 6. Die Patientenliege 6 ist so mit einem Liegensockel 4 verbunden, dass er die Patientenliege 6 mit dem Patienten 3 trägt. Die Patientenliege 6 ist dazu ausgelegt, den Patienten 3 entlang einer Aufnahmerichtung durch die Öffnung 10 der Aufnahmeeinheit 17 zu bewegen. Die Aufnahmerichtung ist in der Regel durch die Systemachse 5 gegeben, um die die Aufnahmeeinheit 17 bei der Aufnahme von Röntgenprojektionen rotiert. In diesem Beispiel ist die Körperachse des Patienten 3 gleich der Systemachse 5. Bei einer Spiral-Aufnahme wird die Patientenliege 6 kontinuierlich durch die Öffnung 10 bewegt, während die Aufnahmeeinheit 17 um den Patienten 3 rotiert und Röntgenprojektionen aufnimmt. Damit beschreiben die Röntgenstrahlen 2 auf der Oberfläche des Patienten 3 eine Spirale.

Der Computertomograph verfügt über eine Datenverarbeitungseinrichtung 12 in Form eines Computers, welcher mit einer Anzeigeeinheit 11, beispielsweise zur graphischen Anzeige von medizinischen Bildaufnahmen, hier in Form von Computer-Tomographieaufnahmen sowie einer Eingabeeinheit 7 verbunden ist. Bei der Anzeigeeinheit 11 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 7 ausgebildet ist. Ein solcher berührungsempfindlicher Bildschirm kann in das bildgebende Gerät integriert oder als Teil eines mobilen Geräts ausgebildet sein. Bei der Eingabeeinheit 7 handelt es sich beispielsweise um eine Tastatur, eine Maus, einen sogenannten "Touch-Screen" oder auch um ein Mikrofon zur Spracheingabe. Die Eingabeeinheit 7 kann auch eingerichtet sein, um Bewegungen eines Nutzers zu erkennen und in entsprechende Befehle zu übersetzen. Mittels Eingabeeinheit 7 kann beispielsweise ein Nutzer eine automatisch durch den Computer 12 durchgeführte Segmentierung einer Außenkontur eines Gewebes in einer interessierenden Körperregion bestätigen. Mittels Eingabeeinheit 7 kann ein Nutzer auch eine automatisch ausgeführte Unterteilung des segmentierten Gewebes in mehrere Gewebebereiche bestätigen, anpassen oder manuell eine Unterteilung in Gewebebereiche vornehmen.

Der Computer 12 steht mit der drehbaren Aufnahmeeinheit 17 zum Datenaustausch in Verbindung. Über die Verbindung 14 werden einerseits Steuersignale für die Datenakquise vom Computer 12 an die Aufnahmeeinheit 17 übertragen, andererseits können für den Patienten 3 aufgenommene Projektionsdaten für eine Bildrekonstruktion mittels gängiger Rekonstruktionsverfahren an den Computer 12 übertragen werden. Die Verbindung 14 ist in bekannter Weise kabelgebunden oder kabellos realisiert.

Die Datenverarbeitungseinrichtung 12 in Form des Computers gemäß diesem Ausführungsbeispiel weist eine lokal angeordnete Recheneinheit 16 auf. Die Recheneinheit 16 ist als Bild- bzw. Bilddatenbearbeitungseinheit ausgestaltet. Sie ist insbesondere eingerichtet, alle im Bezug zu dem erfindungsgemäßen Verfahren stehenden Rechenschritte an einer mit der Aufnahmeeinheit 17 aufgenommenen medizinischen Bildaufnahme durchzuführen. Die medizinische Bildaufnahme kann jedoch auch von einer anderen medizinischen Bildgebungsanlage der Recheneinheit 16 zur Verfügung gestellt werden und muss nicht direkt zeitlich vor einer Weiterverarbeitung des Bilddatensatzes durch die Recheneinheit 16 erfasst worden sein. Beispielsweise kann der Bilddatensatz über einen mobilen, an sich bekannten computerlesbaren Datenträger, über ein Krankenhaus- oder Radiologieinformationssystem (HIS oder RIS) oder über das Internet auf an sich bekannte Weise der Recheneinheit 16 zugeführt werden.

Die Recheneinheit 16 umfasst für die Ausführung des erfindungsgemäßen Verfahrens eine Segmentierungs-Einheit 21 zum Segmentieren der Außenkontur des zu untersuchenden Gewebes in der medizinischen Bildaufnahme. Die Segmentierung erfolgt automatisch oder semi-automatisch, sie kann aber auch, bspw. in Abhängigkeit der Qualität der medizinischen Bildaufnahme bzw. in Abhängigkeit des zu ermittelnden Funktionsparameters vollständig manuell durch einen Nutzer vorgenommen werden. Daneben umfasst die Recheneinheit 16 auch eine Unterteilungseinheit 23, die eingerichtet ist, das segmentierte Gewebe in wenigstens zwei Gewebebereiche zu unterteilen. Die Unterteilungseinheit kann zu diesem Zweck mit dem Computer 12 in Datenverbindung stehen, um Informationen über das Messprotokoll für die Datenakquise bzw. die zugrunde liegende medizinische Fragestellung zu erhalten, um automatisch Anzahl, Größe und/oder Lage oder dergleichen für die Gewebebereiche festzulegen. Daneben umfasst die Recheneinheit 16 auch eine Ermittlungseinheit 22, die eingerichtet ist, für jeden der Gewebebereiche einen Funktionsparameter betreffend die lokale Gewebefunktion zu ermitteln. Dazu wertet die Ermittlungseinheit 22 auf Bildelement-Ebene, also Pixel- oder Voxel-basiert, enthaltene Bildinformation, insbesondere Intensitätswerte, aus und überführt diese für jeden der Gewebebereiche in einen Wert für den betrachteten Funktionsparameter. In Abhängigkeit des zu ermittelnden Funktionsparameters kann eine unterschiedliche Analyse-Vorschrift bzw. Analyse-Funktion zur Auswertung in einem Speicher, insbesondere einem Speicher der Datenverarbeitungsanlage 12 (nicht dargestellt), hinterlegt sein, auf welche die Ermittlungseinheit 22 Zugriff hat und die passende Vorschrift für die Bildanalyse auswählt.

Die Recheneinheit 16 kann mit einem computerlesbaren Datenträger 13 zusammenwirken, insbesondere um durch ein Computerprogramm mit Programmcode ein erfindungsgemäßes Verfahren durchzuführen. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 16, und damit auch ihre Sub-Komponenten, kann in Form von Hard- oder in Form von Software ausgebildet sein. Beispielsweise ist die Recheneinheit 16 als ein sogenanntes FPGA (Akronym für das englischsprachige "Field Programmable Gate Array") ausgebildet oder umfasst eine arithmetische Logikeinheit. Einzelne oder alle Sub-Komponenten können alternativ dezentral angeordnet sein, z.B. können einzelne Rechenschritte des Verfahrens in einem zentralen Rechenzentrum einer medizinischen Dienstleistungseinrichtung, z.B. ein Krankenhaus, oder in der Cloud ausgeführt werden. Hierbei sind insbesondere Daten- und Patientenschutz beim Datenaustausch zu berücksichtigen.

In der hier gezeigten Ausführungsform ist in einem Speicher der Datenverarbeitungsanlage 12 wenigstens ein Computerprogramm gespeichert, welches alle Verfahrensschritte des erfindungsgemäßen Verfahrens durchführt, wenn das Computerprogramm auf dem Computer 12 ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem als dem Computer 12 gespeichert sein. Beispielsweise kann der Computertomograph so ausgelegt sein, dass der Computer 12 das Computerprogramm zum Ausführen des erfindungsgemäßen Verfahrens über ein Intranet oder über das Internet in seinen internen Arbeitsspeicher lädt.

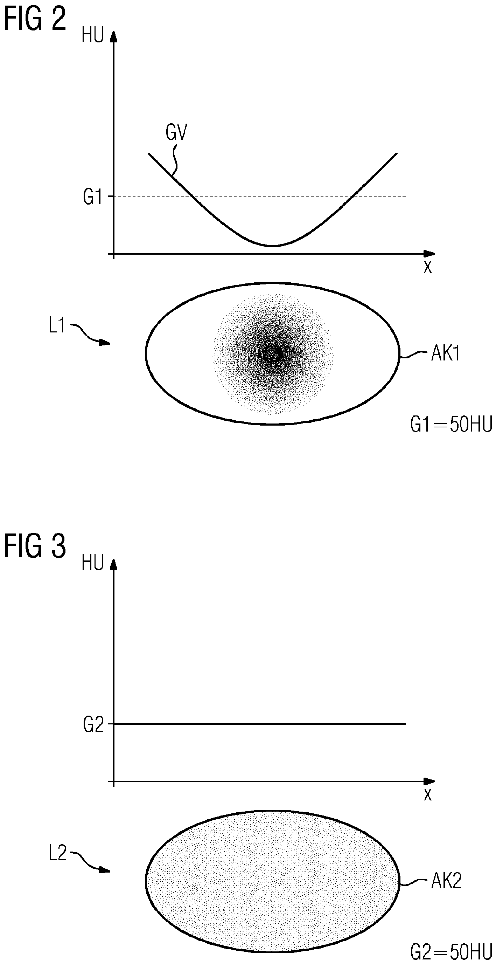

**Figur 2** zeigt beispielhaft eine Läsion L1 in einer medizinischen Bildaufnahme gemäß dem Stand der Technik. Bei der Läsion kann es sich bspw. um tumorartiges Gewebe handeln. Die medizinische Bildaufnahme darstellend die Läsion L1 entspricht einer Computer-Tomographie-Schichtaufnahme, die unter Zufuhr von jodhaltigem Kontrastmittel akquiriert wurde. **Figur 3** zeigt exemplarisch eine Läsion L2, die sich in Form und Größe nicht von der Läsion L1 unterscheidet und die der Einfachheit halber und zum Zwecke der Veranschaulichung mit der gleichen Aufnahmetechnik und Prozedur abgebildet wurde. Beide Läsionen L1 und L2 wurden mittels bekannter Segmentierungsalgorithmen segmentiert, die Außenkontur AK1, AK2 ist jeweils bekannt. Umliegendes Gewebe wird zur Vereinfachung nicht dargestellt. Mittels bekannter Befundungsmechanismen kann für jede Läsion unter Berücksichtigung aller von der Außenkontur AK1, AK2 umfassten Bildelementwerte bzw. hier Pixeleinträge ein mittlerer Graustufenwert G1, G2 (in Houndsfield Units HU) repräsentierend eine Röntgenabsorption ermittelt werden. Dies entspricht einem mittleren Iod-Kontrast bzw. einem mittleren Iod-Gehalt für jede der Läsionen. Erkennbar ist, dass Läsion L1 in ihrem Zentrum einen dunkleren Bereich aufweist, der nach außen hin kontinuierlich an Helligkeit zunimmt. Diese räumliche Graustufen-Verteilung GV ist in Figur 2A in dem über der Läsion L1 angeordneten Diagramm beispielhaft verdeutlicht. Mit anderen Worten ist im Vergleich zu den äußeren Randbereichen im Inneren von Läsion L1 zur Zeit der Bilddatenmessung nur wenig Kontrastmittel angereichert worden, was auf eine verminderte Durchblutung des Läsionsinneren hindeutet. Im Gegensatz dazu zeigt Läsion L2 eine im Wesentlichen homogene Graustufenverteilung, was auf eine gleichmäßige Kontrastmittelverteilung hindeutet. Die ermittelten, mittleren Graustufenwerte G1, G2 können für beide Läsionen L1 und L2 gleich oder nahezu gleich sein. Eine profunde Differenzierung eines Durchblutungszustandes bzw. einer Kontrastmittelverteilung der beiden Läsionen L1 und L2 ist anhand der bekannten Vorgehensweise nicht bzw. nur qualitativ möglich. Eine dedizierte Entscheidung, ob bspw. Läsion L1 auf eine gewählte Therapieform anspricht ist, anhand der beschriebenen, bekannten Vorgehensweise nicht möglich, da lokale Unterschiede der Graustufe innerhalb der Läsion 'weggemittelt' werden.

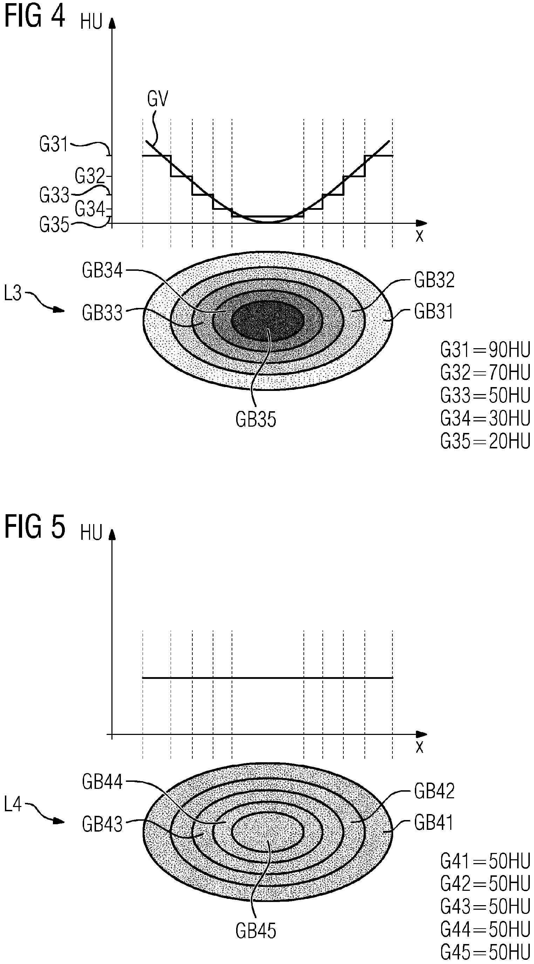

**Figuren 4** und **5** zeigen beispielhaft Läsionen L3 und L4, je in einer medizinischen Bildaufnahme gemäß Ausführungsbeispielen der Erfindung. Die medizinischen Bildaufnahmen darstellend die Läsionen L3, L4 entsprechen ebenfalls Computer-Tomographie-Schichtaufnahmen, die unter Zufuhr von jodhaltigem Kontrastmittel akquiriert wurden. Läsion L4 sei ebenfalls in Form und Größe gleich zu Läsion L3. Entsprechend Läsion L1 weist Läsion L3 in ihrem Zentrum einen dunkleren Bereich auf, der, vergleichbar zu der Situation bei Läsion L1 ein Indikator für eine verminderte Kontrastmittelaufnahme ist. Diese Graustufen-Verteilung GV ist beispielhaft im Diagramm über Läsion L3 in Figur 3A gezeigt. Läsion L4 zeigt wie Läsion L2 eine gleichmäßige Graustufen-Verteilung. Die Außenkonturen AK3, AK4 sind mittels Segmentierung ebenfalls bekannt. Umliegendes Gewebe wird zur Vereinfachung nicht dargestellt. Erfindungsgemäß werden die Läsionen L3, L4 in fünf Gewebebereiche GB31, GB32, GB33, GB34, GB35 und GB41, GB42, GB43, GB44, GB45 unterteilt. Die Unterteilung, also die Definition von Größe, Lage, Anordnung und/der Anzahl der Gewebebereiche erfolgt bevorzugt automatisch und/oder unter Berücksichtigung von Größe, Position oder, sofern bekannt, Art bzw. vermuteter Art der Läsion. Zudem kann basierend auf der medizinischen Fragestellung auch ein im Folgenden zu ermittelnder Funktionsparameter FP oder die verwendete Bildaufnahmetechnik die Unterteilung beeinflussen. Vorliegend wurde eine zwiebelschicht-artige Einteilung vorgenommen, bei der ein jeweils weiter außen liegender Gewebebereich, bspw. GB31 einen innenliegenden Gewebebereich, bspw. GB32 vollständig einschließt. Die Außenkonturen der einzelnen Gewebebereiche sind dabei morphologisch identisch zu der Außenkontur AK3, AK4 der Läsionen L3, L4 gewählt worden. Im Folgenden wird ein Funktionsparameter FP für jeden der einzelnen Gewebebereiche GB31, GB32, GB33, GB34, GB35 und GB41, GB42, GB43, GB44, GB45 aus der medizinischen Bildaufnahme abgleitet. In diesem Beispiel wird für jede Läsion L3, L4 für jeden ihrer Gewebebereiche GB31, GB32, GB33, GB34, GB35 und GB41, GB42, GB43, GB44, GB45 aus den jeweils umfassten Pixeleinträgen ein mittlerer Graustufenwert bzw. Jod-Kontrast G31, G32, G33, G34, G35 bzw. G41, G42, G43, G44, G45 ermittelt. Die gemittelten Jod-Kontrast-Werte geben einen Aufschluss über eine lokale Gewebefunktion GF bzw. geben ein Maß für die Gewebefunktion GF an, in diesem Fall eine Gewebedurchblutung, veranschaulicht durch die Graustufen-Verteilung GF. Mit anderen Worten wird erfindungsgemäß eine Erhöhung der räumlichen Auflösung einer Gewebefunktion GF erreicht. Während mit Bezug zu Läsion L4 für alle ihre fünf Gewebebereiche GB41, GB42, GB43, GB44, GB45 ein im Wesentlichen konstanter Graustufenwert G41=G42=G43=G44=G45=50HU ermittelt wurde, zeigt die erfindungsgemäße Vorgehensweise für Läsion L3 deutliche Unterschiede im Graustufenwert G31=90HU, G32=70HU, G33=50HU, G34=30HU, G35=10HU zwischen den einzelnen Gewebebereichen. Mittels dieser räumlichen Auflösung und unter Einbeziehung einer erfindungsgemäß ausgewerteten früheren medizinischen Bildaufnahme kann nun bspw. ein Ansprechverhalten auf eine Therapie ermittelt werden.

Die der erfindungsgemäßen Auswertung zugrunde gelegte Bildaufnahme kann einer mit beliebiger Aufnahmetechnik gewonnene Darstellung einer interessierenden Region entsprechen oder sie kann aus mehreren Einzelaufnahmen zusammengesetzt sein. Erfindungsgemäß können auch mehrere Bildaufnahmen ausgewertet werden, insbesondere, wenn funktionelle Informationen in den Bildaufnahmen vorhanden sind.

Es sei abschließend nochmals darauf hingewiesen, dass die Form und Größe der Läsionen und Gewebebereiche zur Veranschaulichung nur exemplarisch und insbesondere nicht maßstabsgetreu gewählt wurden. Beliebige andere Formen des untersuchten Gewebes sind möglich.

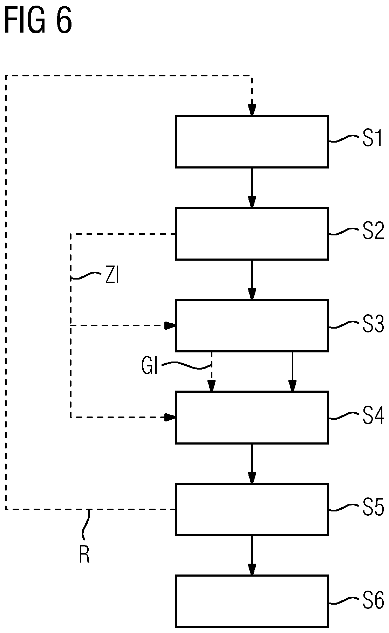

**Figur 6** zeigt eine schematische Darstellung eines erfindungsgemäßen Verfahrens gemäß einem Ausführungsbeispiel der vorliegenden Erfindung. Schritt S1 umfasst das Empfangen wenigstens einer medizinischen Bildaufnahme B einer interessierenden Region eines Untersuchungsobjektes 3, bspw. einer Computertomographie-Aufnahme oder einer MRT-Aufnahme in einer Recheneinheit 16. Die medizinische Bildaufnahme B kann von einer Aufnahmeeinheit 17 zeitlich korreliert zu der erfindungsgemäßen Bildauswertung erfasst werden oder, wenn sie bereits vorher erfasst wurde, aus einem lokalen oder entfernten Speicher, bspw. einen PACS-System eines Krankenhauses in die Recheneinheit 16 geladen werden. Alternativ kann die medizinische Bildaufnahme B erzeugt werden, indem Bildinformation von wenigstens zwei verschiedenen Aufnahmen der interessierenden Region zusammengefügt werden. Hierbei können gängige Bildverarbeitungsverfahren, wie bspw. eine Materialzerlegung, Bildkorrekturverfahren wie bspw. Rauschunterdrückung, Registrierungsverfahren oder dergleichen zum Einsatz kommen, sofern erforderlich. In einem Schritt S3 wird eine Außenkontur eines in der medizinischen Bildaufnahme enthaltenen, zu untersuchenden Gewebes, bevorzugt eine Läsion, in der Recheneinheit 16, insbesondere der Segmentierungseinheit 21, segmentiert. Mit anderen Worten erfolgt eine Einteilung von einzelnen Bildelementen zu dem zu untersuchenden Gewebe bzw. zu umliegendem Gewebe. Bevorzugt kommen dabei Bildelement- bzw. Kantenbasierte Segmentierungsverfahren zum Einsatz, die an sich bekannt sind. In einem optionalen Schritt S2 können für die Segmentierung bzw. vor der Segmentierung durch die Recheneinheit 16 Zusatzinformationen ZI über die der Untersuchung zu Grunde liegende Fragestellung bzw. einen Anfangsverdacht oder über das für die Bilddatenakquise verwendete Messprotokoll erfasst werden, um zu entscheiden, welche interessierende Körperregion und damit welche Gewebetypen abgebildet wurden und/oder welche in der medizinischen Bildaufnahme abgebildete Struktur bzw. welches Gewebe und/oder mit welcher Qualität segmentiert werden soll. Damit kann bspw. der verwendete Segmentierungsalgorithmus ausgewählt werden. Alternativ kann eine entsprechende Eingabe bezüglich der Zusatzinformation ZI von einem Nutzer auf Abfrage oder initiativ per Nutzerschnittstelle 7, 11 der Recheneinheit 16 zur Verfügung gestellt werden. Nach der Segmentierung ist die Größe, also die Längenausdehnung in verschiedenen Raumrichtungen, das Volumen, die Form der Außenkontur AK oder dergleichen des zu untersuchenden Gewebes bekannt. Auch diese Größeninformation GI kann in Folgeschritten verwendet werden. In einem weiteren Schritt S4 erfolgt eine Unterteilung des segmentierten Gewebes in mehrere Gewebebereiche GB. Bevorzugt werden mehr als zwei, insbesondere vier bis fünf Gewebebereiche GB definiert. Je größer die Anzahl der Gewebebereiche GB, umso größer auch die Aussagekraft des im Folgenden ermittelten Funktionsparameters FP über eine lokale Gewebefunktion GF innerhalb des segmentierten Gewebes. Um einen bestmöglichen Kompromiss zwischen für die medizinische Fragestellung notwendige, räumlicher Auflösung der Gewebefunktion GF und erforderlichen Systemressourcen wie Rechenkapazität oder Bearbeitungszeit zu finden, kann für Schritt S4 neben der Zusatzinformation ZI zu der medizinischen Fragestellung oder dem Messprotokoll (aus Schritt S2) auch eine Größeninformation GI über Größe, Volumen und/oder Form und/oder dergleichen des zu untersuchenden Gewebes in die Definition der einzelnen Gewebebereiche GB einfließen. Dazu kann eine von der Recheneinheit 16 umfasste Unterteilungseinheit 23 mit der Segmentierungseinheit 21 und/oder der Datenverarbeitungseinrichtung 12, die auch die Steuerung einer medizinischen Bildgebungsanlage durchführt, in Datenaustausch stehen. Die einzelnen Gewebebereiche GB sind bevorzugt in einander verschachtelt oder anders ausgedrückt, schichtartig aufgebaut und/oder weisen jeweils morphologisch die gleiche Außenkontur wie das segmentierte Gewebe auf. In diesem Fall kann die Unterteilungseinheit 23 die einzelnen Gewebebereiche mittels morphologischer Operationen definieren, wie bspw. einem Erosions- und/oder Dilatationsoperator. Die Gewebebereiche weisen in diesem Fall einen Abstand von Außenkontur zu Außenkontur bzw. eine radiale Ausdehnung von 5 mm auf.

In einem weiteren Schritt S5 erfolgt ein Ermitteln eines Funktionsparameters FP betreffend eine Gewebefunktion GF für jeden der einzelnen Gewebebereiche GB. Mit anderen Worten wird individuell, also lokal für jeden Gewebebereich GB ein Wert für den Funktionsparameter FP abgeleitet. Aus dieser Auswertung können sich unterschiedliche Funktionsparameter-Werte für die einzelnen Gewebebereiche ergeben, die zusammen betrachtet eine räumliche Verteilung einer Gewebefunktion darstellen, wobei lokale Unterschiede der Gewebefunktion aufgelöst werden können. Die Ermittlung des Funktionsparameters wird erfindungsgemäß von der Ermittlungseinheit 22 übernommen. Diese ist eingerichtet, in der medizinischen Bildaufnahme enthaltene Bildinformation für jeden der einzelnen Gewebebereiche individuell zu extrahieren und zu analysieren. Funktionsparamater können sein: Blutfluss, Iod-Anreicherung, Eisenanreicherung, Calcium-Dichte, Fettanteil. Funktionsparameter können aber auch für eine Texturanalyse typische Parameter wie mittlere Dichte, Momente, Heterogenität, Entropie, fraktale Dimension. Entsprechend kann der Ermittlungsschritt eine Texturanalyse umfassen. Dazu kann die Ermittlungseinheit 22 bevorzugt einen Merkmalsvektor basierend auf Intensitätswerten der Bildelemente in jedem einzelnen Gewebebereich bestimmen. Die Ermittlungseinheit 22 kann ferner bevorzugt maschinelle Lernverfahren einsetzen, um basierend auf Bildelementeinträgen und/oder dem Merkmalsvektor wenigstens einen Funktionsparameter zu erzeugen.

Dazu kann in einem Speicher, lokal oder zentral ein Rechenmodell hinterlegt sein, welches vor einer Ausführung des erfindungsgemäßen Verfahrens durch Anwendung eines maschinellen Lernalgorithmus trainiert werden kann. Das Training erfolgt beispielsweise mittels Probebildern und/oder Eingaben durch einen Nutzer, sodass anschließend der Algorithmus in der Lage ist, eigenständig Merkmale in Bildaufnahmen zu detektieren bzw. Funktionsparameter abzuleiten.

Schritt S6 kann folglich umfassen, für jeden Gewebebereich GB eine Texturmetrik zu erstellen. Die Texturanalyse umfasst dabei für den betrachteten Gewebebereich Bildelement-Intensitätswerte zu analysieren und eine räumliche Verteilung derselben abzuleiten. Die Texturmetrik repräsentiert ein Maß für diese räumliche Verteilung, bspw. handelt es sich bei der Texturmetrik um verschiedene Momente dieser Bildelementintensitäten.

Wie eingangs erwähnt, können mehrere Bildaufnahmen zu einer konstruierten, medizinischen Bildaufnahme zusammengefasst sein, um einen gewünschten Bildinhalt zu erzielen oder aber es liegen mehrere medizinische Bildaufnahmen vor. In manchen Fällen kann Schritt S5 umfassen, eine Texturanalyse basierend auf den mehreren Aufnahmen bzw. den mehreren Bildaufnahmen durchzuführen, insbesondere in Fällen, in denen verschiedene medizinische Bildaufnahmen verschiedenen Aufnahmeaspekten, bspw. zeitlichen Phasen, also verschiedenen Zeitphasen einer Bildaufnahme, entsprechen oder aber verschiedenen detektierten Energiebändern. Die verschiedenen Bildaufnahmen können unter Kontrastmittelgabe akquiriert worden sein oder nicht. Ein Merkmalsvektor kann eine Vielzahl von Texturmetriken für eine Mehrzahl von verschiedenen Teilen bzw. Bereichen des zu untersuchenden Gewebes, insbesondere von mehreren Gewebebereichen umfassen.

Eine Texturmetrik im Sinne der Erfindung kann umfassen: eine mittlere, maximale, minimale Bildelement-Intensität, ein Einheitlichkeitsmaß, eine Entropie im Sinne einer Unregelmäßigkeit über ein Grauwert-Histogramm, eine Standard-Abweichung eines Grauwert-Histogramms, eine Schiefe im Sinne einer Asymmetrie eines Grauwert-Histogramms, eine Wölbung bzw. Flachheit eines Grauwert-Histogramms, ein Energiemaß oder ein Flächenträgheitsmoment (bspw. Pixelwiederholrate und/oder ein Ordnungsmaß), Korrelation (bspw. ein Maß für eine lineare Abhängigkeit von Grauwerten), Lauflängenmatrix (bspw. eine Pixeltextur in einer bestimmte Raumrichtung), Kontrast, Rauheit (bspw. als Maß für eine Kantendichte), Heterogenität (bspw. als Maß für das Vorhandensein von Kanten) oder dergleichen.

Die Texturmetrik wird bevorzugt für jeden der Gewebebereiche GB in der medizinischen Bildaufnahme ermittelt.

Bildelement im Sinne der Erfindung soll sowohl Pixel als auch Voxel umfassen.

Liegen mehrere medizinische Bildaufnahmen vor, so kann erfindungsgemäß das beschriebene Verfahren optional in eine Widerholschleife R abzweigen, wobei das Verfahren mit den Schritten S1 bis S5 so oft durchlaufen wird, bis alle medizinischen Bildaufnahmen, die für eine Ableitung der lokalen Gewebefunktion des zu untersuchenden Gewebes zur Verfügung stehen, ausgewertet wurden. In einem weiteren optionalen Schritt S6 kann ein Vergleich von erfindungsgemäß ermittelten Funktionsparametern erfolgen, wobei die Werte für die Funktionsparameter zu medizinischen Bildaufnahmen ermittelt wurden, die zeitlich versetzt erfasst worden. Bspw. kann ein Vergleich desselben Funktionsparameters für medizinische Bildaufnahmen einer Erstuntersuchung zu medizinischen Aufnahmen einer Folgeuntersuchung zu einem späteren Zeitpunkt erfolgen. Der Vergleich ermöglicht, lokale, also auch kleinste Veränderungen des untersuchten Gewebes über den Beobachtungszeitraum hinweg zu identifizieren. Der Vergleich kann bevorzugt unter Anwendung maschineller Lernverfahren erfolgen.

Durch die Objektivierung der in den medizinischen Bilddaten enthaltenen Zustandsinformation bzgl. des zu untersuchenden Gewebes sowie der beliebig einstellbaren räumlichen Auflösung, die erzielt werden kann, eignet sich das beschriebene Verfahren insbesondere für die Verlaufsbeobachtung von Gewebezuständen, bspw. den Fettgehalt oder die Knochendichte und insbesondere zur Verlaufskontrolle einer neuartigen Tumortherapie unter Verzicht auf bisherige Beurteilungskriterien wie RECIST.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu. Weiterhin schließt die Verwendung der unbestimmten Artikel "ein" bzw. "eine" nicht aus, dass die betreffenden Merkmale auch mehrfach vorhanden sein können. Ebenso ist nicht ausgeschlossen, dass als einzelne Einheiten dargestellte Elemente der vorliegenden Erfindung aus mehreren zusammenwirkenden Teil-Komponenten bestehen, die gegebenenfalls auch räumlich verteilt sein können.

## Patentansprüche

1. Verfahren zum Bestimmen einer lokalen Gewebefunktion (GF) eines Gewebes in einer interessierenden Körperregion eines Untersuchungsobjektes (3), wobei das Gewebe ein Tumorgewebe ist oder eine Läsion unbekannter Beschaffenheit mit Vermutung auf Tumorgewebe umfasst, umfassend die Schritte:
- Segmentieren (S3) einer Außenkontur (AK3, AK4) des Gewebes anhand von wenigstens einer medizinischen Bildaufnahme darstellend die interessierende Körperregion des Untersuchungsobjektes umfassend das Gewebe;
- Unterteilen (S4) des segmentierten Gewebes in wenigstens zwei Gewebebereiche (GB; GB31-GB35, GB41-GB45); und
- Ermitteln (S5) eines Funktionsparameters (FP; G31-G35, G41-G45) betreffend die Gewebefunktion für jeden der wenigstens zwei Gewebebereiche, wobei die wenigstens zwei Gewebebereiche derart schichtartig angeordnet sind, dass ein erster Gewebebereich (GB31, GB32) einen zweiten Gewebebereich (GB32, GB42) vollständig umschließt.

2. Verfahren nach Anspruch 1, wobei die Außenkontur der wenigstens zwei Gewebebereiche jeweils die gleiche Form wie die Außenkontur (AK3, AK4) des segmentierten Gewebes aufweist.

3. Verfahren nach Anspruch 1 oder 2, wobei die Außenkonturen der wenigstens zwei Gewebebereiche einen Abstand zu einander aufweisen, der im Bereich von 0,2 mm bis 2,0 mm liegt.

4. Verfahren nach einem der vorhergehenden Ansprüche, wobei das Unterteilen des segmentierten Gewebes unter Verwendung einer morphologischen Operation erfolgt.

5. Verfahren nach einem der vorhergehenden Ansprüche, wobei das segmentierte Gewebe eine medizinische Läsion (L3, L4) umfasst.

6. Verfahren nach einem der vorhergehenden Ansprüche, wobei der Funktionsparameter wenigstens ein Parameter aus der folgenden Gruppe von Parametern ist: Blutfluß, Iod-Anreicherung, Eisenanreicherung, Calcium-Dichte, Fettanteil.

7. Verfahren nach einem der vorhergehenden Ansprüche, wobei der Funktionsparameter wenigstens ein für Texturanalyse typischer Parameter aus der folgenden Gruppe von Parametern ist: mittlere Dichte, Momente, Heterogenität, Entropie, fraktale Dimension.

8. Verfahren nach Anspruch 7, wobei das Ermitteln eines Funktionsparameters eine Texturanalyse umfasst, wobei die Texturanalyse folgende Schritte umfasst:
- Bestimmen eines Merkmalsvektors basierend auf Intensitätswerten von Bildelementen der medizinischen Bildaufnahme,
- Anwenden eines maschinellen Lernverfahrens auf den Merkmalsvektor, und
- Erzeugen des Funktionsparameters als Ergebnis des maschinellen Lernverfahrens.

9. Verfahren nach einem der vorhergehenden Ansprüche, wobei die medizinische Bildaufnahme eine Bildaufnahme aus der folgenden Gruppe von Bildaufnahmen ist: Single-Energy-Aufnahme, multispektrale Computertomographie-Aufnahme, Perfusions-Computertomographie-Aufnahme, Ultraschall-Aufnahme, Magnetresonanz-Aufnahme und Perfusionsmagnetresonanz-Aufnahme.

10. Recheneinheit (12) zum Bestimmen einer Gewebefunktion (GF) eines Gewebes in einer interessierenden Region eines Untersuchungsobjektes (3), aufweisend Mittel (21, 22, 23) zum Durchführen eines Verfahrens gemäß einem der Ansprüche 1 bis9.

11. Medizinische Bildgebungsanlage mit einer Recheneinheit (12) nach Anspruch 10.

12. Computerprogramm mit Programmcode, um das Verfahren nach einem der Ansprüche 1 bis 9 durchzuführen, wenn das Computerprogramm auf einem Computer ausgeführt wird.

13. Computerlesbarer Datenträger (13) mit Programmcode eines Computerprogramms, um das Verfahren nach einem der Ansprüche 1 bis 9 durchzuführen, wenn das Computerprogramm auf einem Computer ausgeführt wird.

## Claims

1. Method for determining a local tissue function (GF) of tissue in a body region of interest of an examination object (3), wherein the tissue is a tumour tissue or comprises a lesion of an unknown nature suspected of being tumour tissue, comprising the steps:
- segmentation (S3) of an outer contour (AK3, AK4) of the tissue using at least one medical image recording representing the body region of interest of the examination object comprising the tissue;
- subdivision (S4) of the segmented tissue into at least two tissue regions (GB; GB31-GB35, GB41-GB45); and
- ascertaining (S5) a function parameter (FP; G31-G35, G41-G45) relating to the tissue function for each of the at least two tissue regions, wherein the at least two tissue regions are arranged in slices such that a first tissue region (GB31, GB32) completely encloses a second tissue region (GB32, GB42).

2. Method according to claim 1, wherein the outer contour of the at least two tissue regions each has the same shape as the outer contour (AK3, AK4) of the segmented tissue.

3. Method according to claim 1 or 2, wherein the outer contours of the at least two tissue regions have a distance from one another within the range of 0.2 mm to 2.0 mm.

4. Method according to one of the preceding claims, wherein the subdivision of the segmented tissue is performed using a morphological operation.

5. Method according to one of the preceding claims, wherein the segmented tissue comprises a medical lesion (L3, L4).

6. Method according to one of the preceding claims, wherein the function parameter is at least one parameter from the following group of parameters: blood flow, iodine-accumulation, iron accumulation, calcium density, fat content.

7. Method according to one of the preceding claims, wherein the function parameter is at least one typical parameter for texture analysis from the following group of parameters: average density, moments, heterogeneity, entropy, fractal dimension.

8. Method according to claim 7, wherein the ascertaining of a function parameter comprises a texture analysis, wherein the texture analysis comprises the following steps:
- determination of a feature vector based on intensity values of image elements of the medical image recording,
- application of a machine learning method to the feature vector, and
- generation of the function parameter as a result of the machine learning method.

9. Method according to one of the preceding claims, wherein the medical image recording is an image recording from the following group of image recordings: single-energy recording, multispectral computed-tomography recording, perfusion-computed-tomography recording, ultrasound recording, magnetic-resonance recording and perfusion-magnetic-resonance recording.

10. Computing unit (12) for determining a tissue function (GF) of tissue in a region of interest of an examination object (3) having means (21, 22, 23) for carrying out a method according to one of claims 1 to 9.

11. Medical imaging system with a computing unit (12) according to claim 10.

12. Computer program with program code for carrying out the method according to one of claims 1 to 9 when the computer program is executed on a computer.

13. Computer-readable data carrier (13) with program code of a computer program for carrying out the method according to one of claims 1 to 9 when the computer program is executed on a computer.

## Revendications

1. Procédé de détermination d'une fonction (GF) tissulaire locale d'un tissu dans une région du corps à laquelle on s'intéresse d'un objet (3) à examiner, le tissu étant un tissu tumoral ou comprenant une lésion de nature inconnue avec suspection de tissu tumoral, comprenant les stades :
- segmentation (S3) d'un contour (AK3, AK4) extérieur du tissu à l'aide d'au moins un enregistrement d'images médicales représentant la région du corps à laquelle on s'intéresse de l'objet à examiner comprenant le tissu ;
- subdivision (S4) du tissu segmenté en au moins deux régions (GB ; GB31 à GB35, GB41 à GB45) de tissu ; et
- détermination (S5) d'un paramètre (FP ; G31 à G35, G41 à G45) fonctionnel concernant la fonction du tissu pour chacune des au moins deux régions du tissu, les au moins deux régions du tissu étant disposées en couche, de manière à ce qu'une première région (GB31, GB32) du tissu entoure complètement une deuxième région (GB32, GB42) du tissu.

2. Procédé suivant la revendication 1, dans lequel les au moins deux régions du tissu ont chacune la même forme que le contour (AK3, AK4) extérieur du tissu segmenté.

3. Procédé suivant la revendication 1 ou 2, dans lequel les contours extérieurs des au moins deux régions du tissu sont à une distance les uns des autres qui est dans la plage de 0, 2 mm à 2, 0 mm.

4. Procédé suivant l'une des revendications précédentes, dans lequel la subdivision du tissu segmenté s'effectue en utilisant une opération morphologique.

5. Procédé suivant l'une des revendications précédentes, dans lequel le tissu segmenté comprend une lésion (L3, L4) médicale.

6. Procédé suivant l'une des revendications précédentes, dans lequel le paramètre fonctionnel est au moins un paramètre choisi dans le groupe suivant de paramètres : flux sanguin, enrichissement en iode, enrichissement en fer, teneur en calcium, proportion de graisse.

7. Procédé suivant l'une des revendications précédentes, dans lequel le paramètre fonctionnel est au moins un paramètre typique pour l'analyse de texture choisi dans le groupe suivant de paramètres : masse volumique moyenne, couple, hétérogénéité, entropie, dimension fractale.

8. Procédé suivant la revendication 7, dans lequel la détermination d'un paramètre fonctionnel comprend une analyse de texture, l'analyse de texture comprenant les stades suivants :
- détermination d'un vecteur de caractéristique reposant sur des valeurs d'intensité d'éléments d'image de l'enregistrement d'image médicale,
- application d'un procédé d'apprentissage automatique au vecteur de caractéristique, et
- production du paramètre fonctionnel en résultat du procédé d'apprentissage automatique.

9. Procédé suivant l'une des revendications précédentes, dans lequel l'enregistrement d'image médicale est un enregistrement choisi dans le groupe suivant d'images médicales : enregistrement single-energy, enregistrement de tomographie par ordinateur multi-spectrale, enregistrement de tomographie par ordinateur de perfusion, enregistrement par ultrasons, enregistrement par résonance magnétique et enregistrement par résonance magnétique par perfusion.

10. Unité (12) informatique de détermination d'une fonction (GF) tissulaire d'un tissu dans une région à laquelle on s'intéresse d'un objet (3) à examiner, comportant des moyens (21, 22, 23) pour effectuer un procédé suivant l'une des revendications 1 à 9.

11. Installation d'imagerie médicale ayant une unité (12) informatique suivant la revendication 10.

12. Programme d'ordinateur ayant des codes de programme pour effectuer le procédé suivant l'une des revendications 1 à 9, lorsque le programme d'ordinateur est exécuté sur un ordinateur.

13. Support (13) de données déchiffrables par ordinateur ayant un code d'un programme d'ordinateur pour effectuer le procédé suivant l'une des revendications 1 à 9, lorsque le programme d'ordinateur est réalisé sur un ordinateur.
